# EUROPEAN PATENT APPLICATION

(11) **EP 4 763 859 A1**
(43) Date of publication of application: **24.06.2026**
(21) Application number: 24933781.7
(22) Date of filing: 01.11.2024
(51) Int. Cl.: C07K 14/78, C07K 19/00, C12N 15/12, C12N 15/62, C07K 1/34, C07K 1/22, C07K 1/14, C07K 1/36, C12N 15/81, A61K 8/64, A61Q 19/08, A61Q 19/00, A61Q 17/04, A61L 15/32, A61L 15/44, A61L 27/54, A61L 27/60, C12R 1/84

(54) **USE OF RECOMBINANT HUMAN ELASTIN PEPTIDE WITH ANTI-AGING EFFECTS AND COMPOSITION THEREOF**

(30) Priority: 01.04.2024 CN 202410387107
(71) Applicant: Hangzhou Enhe Biotechnology Co., Ltd., Hangzhou, Zhejiang 310017 (CN)
(72) Inventor: SHAO, Qimiao, Hangzhou, Zhejiang 310017 (CN); PANG, Zhenhua, Hangzhou, Zhejiang 310017 (CN); ZHAO, Na, Hangzhou, Zhejiang 310017 (CN); WANG, Xiaoyue, Hangzhou, Zhejiang 310017 (CN); CHEN, Fengyi, Hangzhou, Zhejiang 310017 (CN); LI, Faliang, Hangzhou, Zhejiang 310017 (CN); XU, Haojie, Hangzhou, Zhejiang 310017 (CN)
(74) Representative: Fish & Richardson P.C.
(86) International application number: PCT/CN2024/129480
(87) International publication number: WO 2025/208856

(57) **Abstract**

The present invention relates to the technical field of biology, and disclosed are a recombinant human-derived elastin peptide having anti-aging efficacy and a use thereof. The recombinant human-derived elastin peptide comprises the amino acid sequence set forth in SEQ ID NO: 1, or comprises a fragment of the amino acid sequence set forth in SEQ ID NO: 1, the fragment comprising 30% or more of SEQ ID NO: 1, or comprises a mutant of the amino acid sequence set forth in SEQ ID NO: 1 or a fragment of the amino acid sequence set forth in SEQ ID NO: 1. The method for producing elastin peptide by using a yeast expression system disclosed in the present invention can directionally synthesize a high-efficacy protein fragment having 100% homology with a human-derived protein, and can also achieve low costs, have easy large-scale production, avoid animal sources, and avoid hidden dangers of viruses. The present invention enables the recombinant human-derived elastin to be safely used in cosmetics as an anti-aging and anti-photoaging functional ingredient, and also provides support for use thereof in the field of biomedical materials.

## Description

### CROSS REFERENCE TO RELATED APPLICATIONS

The present application claims priority to Chinese Patent Application No. 202410387107.X filed on April 1, 2024 and having the title of invention "USE OF RECOMBINANT HUMAN ELASTIN PEPTIDE WITH ANTI-AGING EFFECTS AND COMPOSITION THEREOF", the entire contents of which are incorporated herein by reference.

### TECHNICAL FIELD

The present invention relates to the technical field of biology, and in particular to a recombinant human-derived elastin peptide having anti-aging efficacy, and a use thereof.

### BACKGROUND ART

Skin aging refers to a process in which the skin gradually loses elasticity and firmness with age. It is caused by a variety of factors, including natural aging, environmental factors, lifestyle, heredity, etc. With the improvement of people's living standards, skin aging has attracted widespread attention from society. Major features of aged skin include thinning of the epidermis, leveling of the dermal-epidermal junction, and degradation of the dermal extracellular matrix. Elastin, as the second major class of proteins besides collagen in the extracellular matrix, plays an important role in skin aging. Degradation and destruction thereof can cause symptoms such as decreased skin elasticity, sagging skin, wrinkles, etc.

Currently, the main source of elastin is extraction from animal tissue. A tissue or biological sample rich in elastin, such as animal blood vessel, skin, lung tissue, etc., is collected. The tissue is crushed and then treated using a chemical method or a high-temperature treatment method, to remove non-elastin components such as collagen. The treated tissue is then mixed with a solvent, to allow dissolution and extraction under certain temperature conditions, such that the elastin dissolves and is released into the solution. Subsequently, filtration, precipitation, washing and purification are performed, to obtain elastin having a high purity. The extracted elastin maintains a maximum similarity to natural protein in terms of molecular weight and structure. However, immunogenicity, hidden danger of viruses, and unity of the extracted protein sequence associated with the extraction process all represent difficulties for the use of elastin in cosmetics and medical materials. In recent years, quite a number of elastin versions synthesized using gene recombination technology have progressively emerged. For example, CN202110357328.9 uses Saccharomyces cerevisiae to synthesize the key hexapeptide GVGVAP repeat sequence of elastin, the elastin fragment having good biocompatibility, antioxidant properties and reducing properties. For example, CN202211035433.1 describes a method for synthesizing the elastin (VPTGIG)25 repeat sequence using E. coli, the method allowing simple purification and providing a new type for drug carriers. CN202010611388.4 discloses an E. coli-biosynthesized fragment consisting of 3 to 7 tandem repeats of the repeat sequence (VAPGVG)3S of elastin, the fragment being proven to have a high scavenging activity against superoxide free radicals.

However, there are still technical problems in the prior art, such as high cost and unfavorability for large-scale production. Thus, there is an urgent need for a use of a recombinant human-derived elastin peptide having anti-aging efficacy and a composition thereof, in order to solve such problems.

### SUMMARY OF THE INVENTION

The present invention provides a use of a recombinant human-derived elastin peptide having anti-aging efficacy and a composition thereof in order to solve the described problems in the prior art.

Disclosed in the present invention is a recombinant human-derived elastin peptide having anti-aging efficacy, the recombinant human-derived elastin peptide being selected from the following a) to c): a) comprising the amino acid sequence shown in SEQ ID NO: 1; b) comprising a fragment of the amino acid sequence shown in SEQ ID NO: 1, the fragment comprising 30% or more of SEQ ID NO: 1; and c) mutants of a) and b) above.

In preferred technical solutions, the recombinant human-derived elastin peptide comprises the amino acid sequence shown in SEQ ID NO: 21 or includes variant sequences obtained by insertion, substitution or deletion of one or more amino acids on the basis of SEQ ID NO: 21.

In preferred technical solutions, the variant sequences have at least 85%, 90%, 95%, 96%, 97%, 98%, 99% or more identity to SEQ ID NO: 21; preferably, the variants retain anti-aging efficacy equivalent to that of SEQ ID NO: 21.

In preferred technical solutions, the anti-aging efficacy is selected from one or more of the following: 1) promoting the expression of elastin per se; 2) promoting gene expression of collagen IV and/or VII in the dermal-epidermal junction layer; 3) protecting collagen I, etc. from being damaged by ultraviolet rays; 4) increasing or maintaining the water content of skin; 5) improving skin gloss; 6) reducing crow's feet; 7) increasing the L value of skin; 8) increasing skin density; and 9) increasing skin elasticity or firmness.

In preferred technical solutions, the recombinant human-derived elastin peptide consists of the amino acid sequence shown in SEQ ID NO: 21.

In preferred technical solutions, the N-terminus and/or C-terminus of the sequence of the recombinant human-derived elastin peptide comprises a fusion protein sequence.

In preferred technical solutions, the fusion protein sequence is a protein tag.

In preferred technical solutions, the protein tag is one of a His tag, a glutathione thioltransferase tag, a maltose binding protein tag, a SUMO tag, a NusA tag, a TrxA tag, and a DsbA tag.

As preferred technical solutions, the recombinant human-derived elastin peptide is expressed by a host, the host being one of a bacterium, a fungus and a eukaryotic cell.

In preferred technical solutions, the bacterium is one of Escherichia coli, Bacillus subtilis, and Rhodococcus erythropolis; the fungus is one of Pichia pastoris, Saccharomyces cerevisiae, and Aspergillus oryzae; and the eukaryotic cell is one of an insect cell, a CHO cell, a mouse cell, and a human cell.

In preferred technical solutions, the recombinant human-derived elastin peptide is expressed in a host cell.

In preferred technical solutions, the recombinant human-derived elastin peptide is expressed and secreted by the host cell.

In preferred technical solutions, the recombinant human-derived elastin peptide is expressed and secreted, and a precursor protein thereof includes a secretory signal peptide sequence. A secretory signal peptide is a short peptide that guides the transfer of a newly synthesized protein towards a secretory pathway. In a mature protein secreted out of a cell, the secretory signal peptide has been cleaved by a signal peptidase.

In preferred technical solutions, the recombinant human-derived elastin peptide is expressed and excreted by one of Pichia pastoris or Saccharomyces cerevisiae.

In preferred technical solutions, a precursor protein of the recombinant human-derived elastin peptide contains a signal peptide sequence of Saccharomyces cerevisiae mating factor α, the amino acid sequence of the signal peptide of the mating factor α being as shown in SEQ ID NO: 2.

In preferred technical solutions, the gene encoding the recombinant human-derived elastin peptide comprises a fragment encoding the elastin peptide in the cDNA sequence of the human-derived elastin gene and an artificially synthesized gene.

In preferred technical solutions, the recombinant human-derived elastin peptide is expressed and secreted, and the gene thereof encodes a precursor of the recombinant human-derived elastin peptide, as represented by the fact that the gene thereof comprises a nucleotide sequence encoding the signal peptide and a nucleotide sequence encoding the recombinant human-derived elastin peptide.

In preferred technical solutions, the gene sequence encoding the recombinant human-derived elastin peptide is a codon-optimized artificially synthesized gene sequence. Codon optimization is a protocol for redesigning a gene sequence by methods such as avoiding rare codons and utilizing preferred codons, simplifying the secondary structure of mRNA, optimizing a repeat sequence, and adjusting GC content, so as to improve translation efficiency and thus increase the protein expression level.

In preferred technical solutions, the artificially synthesized gene encoding the elastin peptide is obtained by performing codon optimization on a nucleotide sequence encoding the signal peptide and the elastin peptide, and then performing synthesis.

In preferred technical solutions, the artificially synthesized gene encoding the elastin peptide is obtained by performing codon optimization on a nucleotide sequence encoding the signal peptide, the elastin peptide and the C-terminal His tag, and then performing synthesis. In preferred technical solutions, the nucleotide sequence of the artificially synthesized gene encoding the elastin peptide is as shown in SEQ ID NO: 3 or 31.

In preferred technical solutions, the expression of the gene encoding the recombinant human-derived elastin peptide or a precursor protein thereof is initiated by a promoter. Promoters comprise an inducible promoter and a constitutive promoter.

In preferred technical solutions, the expression of the gene encoding the recombinant human-derived elastin peptide or a precursor protein thereof is initiated by an inducible promoter.

In preferred technical solutions, the expression of the gene encoding the recombinant human-derived elastin peptide or a precursor protein thereof is initiated by a constitutive promoter.

In preferred technical solutions, the gene encoding the recombinant human-derived elastin peptide or a precursor protein thereof is introduced into a host cell.

In preferred technical solutions, the gene encoding the recombinant human-derived elastin peptide or a precursor protein thereof is located on a mobile genetic element. In preferred technical solutions, the mobile genetic element is a plasmid.

In preferred technical solutions, the gene encoding the recombinant human-derived elastin peptide or a precursor protein thereof is integrated into the genome of a host.

Further disclosed in the present invention is an isolated nucleic acid encoding the recombinant human-derived elastin peptide of the present invention.

In preferred technical solutions, the isolated nucleic acid is codon-optimized.

In preferred technical solutions, the isolated nucleic acid is codon-optimized for E. coli or yeast.

In preferred technical solutions, the isolated nucleic acid is an artificially synthesized gene.

In preferred technical solutions, the isolated nucleic acid encodes a precursor of the recombinant human-derived elastin peptide, as represented by the fact the gene thereof comprises a nucleotide sequence encoding a signal peptide and a nucleotide sequence encoding the recombinant human-derived elastin peptide.

In preferred technical solutions, the isolated nucleic acid encodes a precursor of the recombinant human-derived elastin peptide, as represented by the fact the gene thereof comprises a nucleotide sequence encoding a signal peptide, a nucleotide sequence encoding the recombinant human-derived elastin peptide, and a nucleotide sequence encoding a C-terminal His tag.

In preferred technical solutions, the isolated nucleic acid is obtained by performing codon optimization on the nucleotide sequences encoding the signal peptide, the elastin peptide and the C-terminal His tag, and then performing synthesis.

In preferred technical solutions, the isolated nucleic acid includes a nucleotide sequence as shown in SEQ ID NO: 3 or 31.

Further disclosed in the present invention is a vector including the isolated nucleic acid of the present invention.

In preferred technical solutions, the vector is a eukaryotic vector or a prokaryotic vector.

Further disclosed in the present invention is a host cell including the isolated nucleic acid of the present invention or the vector of the present invention.

In preferred technical solutions, the host cell is a bacterium, a fungus, or a eukaryotic cell.

In preferred technical solutions, the bacterial host is one of Escherichia coli, Bacillus subtilis, and Rhodococcus erythropolis.

In preferred technical solutions, the fungal host is one of Pichia pastoris, Saccharomyces cerevisiae, and Aspergillus oryzae.

In preferred technical solutions, the eukaryotic cell is one of an insect cell, a CHO cell, a mouse cell, and a human cell.

Further disclosed in the present invention is a method for preparing a recombinant human-derived elastin peptide having anti-aging efficacy from a fermentation culture, comprising the following steps:
1) Performing fermentation of a host cell comprising a gene encoding the recombinant human-derived elastin peptide or a precursor protein thereof; and
2) Performing protein purification, to obtain the recombinant human-derived elastin peptide from the fermentation culture.

For a fermentation culture involving intracellular expression, the recombinant human-derived elastin peptide is prepared from a cell lysate; for a fermentation culture involving expression and secretion, the recombinant human-derived elastin peptide is prepared from a fermentation supernatant.

The purification preparation of the protein can be carried out by conventional methods in the art, for e.g., the method described by Wingfield in "Overview of the purification of recombinant proteins" (Curr Protoc Protein Sci 2015 80:6.1.1-6.1.35. doi: 10.1002/0471140864.ps0601s80).

In preferred technical solutions, the following method is used to perform protein purification: for a fermentation culture involving intracellular expression, performing lysing, centrifuging the lysate in a low-temperature centrifuge, and retaining the supernatant, or for a fermentation culture involving expression and secretion, centrifuging the fermentation culture in a low-temperature centrifuge, and retaining the supernatant;
Filtering the obtained supernatant using a 0.45 µm filter membrane, and then performing protein purification using an affinity chromatography column, to obtain the recombinant human-derived elastin peptide having anti-aging efficacy.

As a preferred technical solution, performing protein purification using an affinity chromatography column comprises the following steps:
S1, Flowing a sample through an Ni column at a rate of 1 ml/min;
S2, Equilibrating the column using Buffer A;
S3, Eluting using 20 mM, 300 mM and 500 mM imidazole; and
S4, Performing an SDS-PAGE gel analysis on the eluted sample portions for the presence of the target protein.

Further disclosed in the present invention is a composition of a recombinant human-derived elastin peptide composition having anti-aging efficacy, the composition comprising the recombinant human-derived elastin peptide in an amount of 0.01% to 5%, and the remainder being formulation agents.

In preferred technical solutions, the formulation agents comprise the following ingredients in percentages by mass: 0.05 to 1 part by weight of a chelating agent, 0.1 to 0.5 parts by weight of a thickener, 3.0 to 6.0 parts by weight of a polyol, 2 to 5 parts by weight of a vegetable fat, 1 to 3 parts by weight of a high-melting point fatty compound, 3 to 20 parts by weight of a skin moisturizer, 2 to 10 parts by weight of an emulsifier, and 1 to 3 parts by weight of a preservative, the system being adjusted to a pH of 6.0 to 7.0, and being made up to 100 parts by weight with water.

Further disclosed in the present invention is a composition of a recombinant human-derived elastin peptide composition having anti-aging efficacy, the composition comprising the recombinant human-derived elastin peptide and auxiliary agents; preferably, the recombinant human-derived elastin peptide comprises the amino acid sequence shown in SEQ ID NO:21; preferably, the composition is an emulsion, an aqueous agent, or an aqueous solution.

In preferred technical solutions, the dosage form of the composition is an aqueous agent, and the auxiliary agents comprise a solvent, a humectant, a penetration enhancer, and a solubilizer. Preferably, the solvent is water; preferably, the humectant comprises propylene glycol; preferably, the penetration enhancer comprises tetrahydropiperine and/or tridecapeptide-1; and preferably, the solubilizer comprises PEG-40 hydrogenated castor oil.

In preferred technical solutions, the dosage form of the composition is an emulsion, and the auxiliary agents comprise one or more selected from the group consisting of a solvent, a chelating agent, a thickener, an emulsifier, an emollient, a humectant, a penetration enhancer, and a pH regulator. Preferably, the solvent is water; preferably, the chelating agent is disodium EDTA; preferably, the thickener comprises one or more of Carbomer, xanthan gum and polyacrylate crosspolymer-6; preferably, the emulsifier is selected from methyl glucose sesquistearate and/or PEG-20 methyl glucoside sesquistearate; preferably, the emollient is selected from dimethicone and/or triethylhexanoin; preferably, the penetration enhancer is selected from one or more of phytol, tetrahydropiperine and tridecapeptide-1; preferably, the humectant comprises propylene glycol.

Further disclosed in the present invention is a use of the composition of a recombinant human-derived elastin peptide having anti-aging efficacy in the preparation of skin care products, skin repair dressings, implants, artificial skin, medical devices, and biomaterials.

The present invention has the following advantages:
1) The present invention uses Pichia pastoris fermentation to synthesize a functional fragment of human-derived elastin. The fragment can significantly promote the expression of elastin per se, and can also significantly promote the synthesis of genes for collagen IV and VII in the dermal-epidermal junction layer. An anti-UV experiment showed that the recombinant human-derived elastin fragment can protect collagen I, etc. from being damaged by ultraviolet rays. A clinical experiment confirmed that an emulsion containing the recombinant elastin fragment can significantly improve the density and elasticity of the dermis, and at the same time, the skin moisturization and skin roughness are also significantly improved.
   A transdermal experiment found that the human-derived elastin peptide of the present invention also has good transdermal absorption performance.
2) The present invention uses gene recombination technology to access the genome of Pichia pastoris, utilizes the expression system of Pichia pastoris to synthesize elastin with high efficiency, and then performs separation and purification, to obtain the recombinant elastin having high purity.

The method can directionally synthesize a high-efficacy fragment having 100% homology with a human-derived protein, and can also achieve low cost, have easy large-scale production, avoid animal sources, avoid hidden dangers of viruses. The present invention enables the recombinant human-derived elastin to be safely used in cosmetics as an anti-aging and anti-photoaging functional ingredient, and also provides support for use thereof in the field of biomedical materials.

### BRIEF DESCRIPTION OF THE DRAWINGS

FIG. 1 is a map of an integration plasmid pELN for the recombinant human-derived elastin peptide (SEQ ID NO: 1) of the present invention, in which "elastin peptide" stands for a coding sequence of the recombinant human-derived elastin peptide having the sequence of SEQ ID NO: 1.
FIG. 2 is an electrophoretic image showing insertion of the elastin peptide integration plasmid pELN1 into the genome of a transformant as verified by PCR in an example of the present invention.
FIG. 3 is an SDS-PAGE image of the recombinant elastin peptide (SEQ ID NO: 1) after fermentation and purification according to an example of the present invention.
FIG. 4 is a graph showing the promoting effect of the recombinant elastin of the present invention on the gene expression of elastin in fibroblasts, wherein, as the concentration of the recombinant elastin (ELN) added increased, the gene expression of elastin significantly increased and reached a high value at 15 ppm; in FIG. 4, ELN on the abscissa stands for the recombinant elastin peptide, and ELN on the ordinate stands for the elastin synthesized by fibroblasts, and NT in FIG. 4 indicates no treatment.
FIG. 5 is a graph showing the promoting effect of the recombinant elastin of the present invention on the gene expression of collagen VII, wherein, as the concentration of the recombinant elastin (ELN) added increased, the gene expression of collagen VII significantly increased in a concentration-dependent manner; NT in FIG. 5 indicates no treatment.
FIG. 6 is a graph showing the promoting effect of the recombinant elastin of the present invention on the gene expression of collagen IV, wherein, as the concentration of the recombinant elastin (ELN) added increased, the gene expression of collagen IV significantly increased in a concentration-dependent manner; NT in FIG. 6 indicates no treatment.
FIG. 7 is a comparison graph of UVA irradiation according to the present invention, wherein the gene expression of collagen I in fibroblasts after UVA irradiation was significantly decreased, whereas, in the fibroblasts treated with the recombinant elastin (ELN), the gene expression of collagen I was restored, demonstrating that the recombinant elastin had significant protection efficacy against UVA.
FIG. 8 is a comparison graph of skin moisture content after 14 days of use of an emulsion formulation comprising 60 ppm of the recombinant elastin in an experiment of the present invention, wherein 2% ELN (i.e., the product of Example 3 of the present invention) achieved a skin moisture content 100% higher than that of the control group (placebo), and the effect was more significant as the use time increased.
FIG. 9 is a comparison graph comparing skin gloss after 14 days of use of an emulsion formulation comprising 60 ppm of the recombinant elastin in an experiment of the present invention, wherein 2% ELN (i.e., the product of Example 3 of the present invention) achieved a significant increase in skin gloss, and as the use time increased, i.e., after two months, the skin gloss could achieve a two-fold improvement.
FIG. 10 is a comparison graph of crow's feet after 14 days of use of an emulsion formulation comprising 60 ppm of the recombinant elastin in an experiment of the present invention, wherein 2% ELN (i.e., the product of Example 3 of the present invention) had a very significant effect in reducing crow's feet, and as the use time increased, the effect was more significant.
FIG. 11 is a comparison graph of skin L value after two months of use of an emulsion formulation comprising 60 ppm of the recombinant elastin in an experiment of the present invention, wherein 2% ELN (i.e., the product of Example 3 of the present invention) achieved a significant increase in the skin L value in comparison to the control group (placebo), indicating that the formulation had a certain whitening efficacy.
FIG. 12 is a map of integration plasmid pELN2 for the recombinant human-derived elastin peptide (SEQ ID NO: 21) of the present invention, in which "elastin truncated peptide" stands for a coding sequence of the recombinant human-derived elastin peptide having the sequence of SEQ ID NO: 21.
FIG. 13 is an electrophoretic image showing insertion of the recombinant human-derived elastin peptide integration plasmid pELN2 into the genome of a transformant, as verified by PCR, in an example of the present invention.
FIG. 14 is an SDS-PAGE image of the fermentation supernatant of the recombinant human-derived elastin peptide (SEQ ID NO: 21) according to an example of the present invention.
FIG. 15 is an elution curve of an FITC-labeled recombinant human-derived elastin peptide fragment in an example of the present invention.
FIG. 16 is a fluorometric quantification standard curve of an FITC-labeled recombinant human-derived elastin peptide in an example of the present invention.
FIG. 17 shows the distribution of FITC-elastin in different dosage forms in the skin as determined using CLSM in an example of the present invention: A, an aqueous solution of the recombinant human-derived elastin peptide; B, an emulsion of the recombinant human-derived elastin peptide; and C, an aqueous agent of the recombinant human-derived elastin peptide.
FIG. 18 is a comparison diagram of dermal density after 14 days, 28 days and 56 days of use of an emulsion formulation containing 60 ppm of the recombinant elastin peptide of the present invention (compared with a control group).
FIG. 19 is a comparison diagram of skin elasticity after 14 days, 28 days and 56 days of use of an emulsion formulation containing 60 ppm of the recombinant elastin peptide of the present invention (compared with a control group).
FIG. 20 is a comparison diagram of skin firmness after 14 days, 28 days and 56 days of use of an emulsion formulation containing 60 ppm of the recombinant elastin peptide of the present invention (compared with a control group).
FIG. 21 is a comparison diagram of skin roughness after 14 days, 28 days and 56 days of use of an emulsion formulation containing 60 ppm of the recombinant elastin peptide of the present invention (compared with a control group).

### DETAILED DESCRIPTION OF THE INVENTION

In order to make up for the above disadvantages, the present invention provides a use of a recombinant human-derived elastin peptide having anti-aging efficacy and a composition thereof so as to solve the described problems in the prior art.

### Definitions of terms

As used herein, the term "peptide" or "polypeptide" refers to a polymer of amino acid residues. A "peptide" or "polypeptide" may be modified (e.g., phosphorylated or glycosylated) or unmodified. A "polypeptide" may comprise a "conservative substitution". The "conservative substitution" in relation to an amino acid sequence refers to the replacement of an amino acid residue by a different amino acid residue having a side chain with similar physicochemical properties. For example, the conservative substitution may be made between amino acid residues having a hydrophobic side chain, between amino acid residues having a neutral hydrophilic side chain, between amino acid residues having an aromatic side chain, between amino acid residues having an acidic side chain, or between amino acid residues having a basic side chain. As is known in the art, a conservative substitution typically would not cause a significant change in the conformational structure of a protein, and thus the biological activity of the protein may be retained.

As used herein, "vector" refers to a DNA molecule that undertakes the function of transferring a DNA fragment (a gene of interest) to a recipient cell in the context of genetic engineering recombinant DNA technology. Vectors can be categorized into cloning vectors and expression vectors. Cloning vectors are mainly used to clone and amplify a DNA fragment. Cloning vectors mainly include plasmid vectors, phage vectors, phagemid vectors, and virus vectors. Expression vectors have, in addition to the basic elements of cloning vectors, control elements necessary for transcription and translation, such as a promoter and a terminator.

As used herein, "integration plasmid" refers to a DNA sequence comprising a selectable marker and at least one gene expression cassette of interest. The integration plasmid is linearized before being transformed into a yeast, which allows insertion of its sequence into a genomic region of the yeast.

As used herein, the term "emulsion" generally refers to a dispersion system composed of two or more mutually immiscible liquids, in which one liquid is dispersed in the form of small droplets in the other liquid. The diameter of these droplets is typically between 100 nanometers and 10 micrometers. The formation of an emulsion generally needs to be aided by an emulsifier, which can reduce interfacial tension, to enable two liquids which are otherwise incompatible to coexist stably. Common emulsions include water-in-oil (W/O) emulsions and oil-in-water (O/W) emulsions, among others.

As used herein, the term "aqueous agent" generally refers to a homogeneous liquid mixture formed by dissolving or dispersing other substances in water as a main solvent. The solute in an aqueous agent may be various inorganic compounds, organic compounds, etc. Water, as a solvent, has good solubility and dispersibility, and can cause the solute to be uniformly distributed in it. The properties of an aqueous agent depend on the type and concentration of the solute and the properties of the solvent (water).

As used herein, the term "chelating agent" is a class of organic compounds capable of forming a complex by coordinating with a metal ion. The binding ability with the metal ion is stronger than that with a water molecule, and a relatively stable complex can be formed. The structure of a chelating agent generally comprises a plurality of functional groups capable of forming a complex with a metal, such as carboxyl group, alcoholic hydroxyl group, thiol group, amine group, etc. Commonly used chelating agents include disodium EDTA, diethylenetriaminepentaacetic acid (DTPA), n,n,n',n'-tetra(carboxymethyl)ethylenediamine, NTA, ethylenediaminetetraacetic acid (EGTA), N,N,N',N'-tetrakis(2-pyridinecarboxylate)ethylenediamine (TPEN), etc.

As used herein, the term "thickener", also known as gelling agent, is a substance that can increase the viscosity of a latex or a liquid. Common thickening agents include Carbomer, xanthan gum, polyacrylate crosspolymer-6, sodium polyacrylate, polyvinylpyrrolidone, methylcellulose, hydroxyethylcellulose, etc.

As used herein, the term "emulsifier" is a substance that is capable of forming a stable emulsion from a mixed liquid of two or more immiscible components. Common cosmetic emulsifiers include PEG-20 methyl glucoside sesquistearate, methyl glucose sesquistearate, steareth-2, steareth-21, ceteareth-2, ceteareth-21, beheneth-25, PEG-7 olive oil sodium carboxylate, ceteareth-6 olive oil ester, cetearyl, PEG-7 hydrogenated castor oil, PEG-40 castor oil, polyglyceryl-2 dipolyhydroxystearate, etc.

As used herein, the term "emollient" refers to a cosmetic component that helps maintain the soft, pliable, and smooth appearance of the skin. Common emollients include polydimethylsiloxane (PDMS), triethylhexanoin, etc.

As used herein, the term "humectant" generally refers to a substance that is capable of absorbing moisture from the surrounding environment or preventing moisture loss, thereby increasing or maintaining the moisture content of a system. Humectants generally have hydrophilic functional groups, such as a hydroxyl group, a carboxyl group, an amide group, etc., which are capable of forming hydrogen bonds or other types of bonds with water molecules, thereby effectively capturing and retaining moisture. Common humectants include polyols (such as glycerol and propylene glycol), natural moisturizing factors (such as amino acids, urea, etc.), hyaluronic acid, etc., which are widely used in the fields of cosmetics, medicine, food, etc.

As used herein, the term "penetration enhancer" generally refers to a substance that can increase the penetration of drugs or other active ingredients through a biological barrier such as skin and mucosa. Penetration enhancers can alter the physical structure of the biological barrier, e.g., increase its porosity, decrease its lipid orderliness, etc., thereby facilitating the penetration of substances. Common penetration enhancers include surfactants, alcohols, fatty acids, etc., which find important applications in the fields of medicine, cosmetics, etc. Penetration enhancers used in the present invention include phytol, tetrahydropiperine, tridecapeptide-1, etc. As used herein, the term "skin moisturizer" is a class of mild lipophilic substances that can make the skin softer and more pliable, which not only have the effect of lubricating the skin, but also can cover the skin, reduce water evaporation from the skin surface, diffuse water from the basal tissue to the stratum corneum, induce further hydration of the stratum corneum, and preserve the skin's own moisture, thereby achieving a skin moisturizing effect. There are a wide variety of moisturizers, including various oils, fats and waxes, alkanes, fatty acids, fatty alcohols and esters thereof, natural animal and vegetable oils, fatty acid glycerides, etc.

In order that the technical means, inventive features, and objectives and effects achieved by the present invention can be easily and clearly understood, the present invention will be further described below in conjunction with specific examples.

### Examples

### Example 1. Construction of an elastin peptide integration plasmid expression system and fermentation production

Construction of an integration plasmid Elastin peptide (SEQ ID NO: 1) is a fragment (amino acids 85 to 184) of human-derived elastin (UniProt ID P15502), obtained by performing screening of elastin.

For ease of purification, a His tag (-GSSHHHHHH, SEQ ID NO: 30) was attached to the C-terminus. This peptide fragment was designed to be expressed in a secretory manner by the yeast Komagataella phaffii. The signal peptide (SEQ ID NO: 2) of mating factor α of the yeast Saccharomyces cerevisiae was placed upstream of the elastin peptide sequence, to guide secretion. In order to improve expression efficiency, the product gene sequence was designed and optimized using codon optimization technology.

The specific method was as follows: The nucleotide sequence encoding the signal peptide and the C-terminal His-tagged elastin peptide was codon optimized using the "Codon Optimization Tool" on the website of Integrated DNA Technologies, Inc. The codon optimized gene sequence (SEQ ID NO: 3) was subsequently synthesized by BGI Tech Solutions (Beijing Liuhe) Co., Ltd.

An integration plasmid was used to integrate an elastin peptide expression cassette into the yeast genome. The elastin peptide expression cassette comprises the methanol-inducible promoter of the AOX1 gene (SEQ ID NO: 4, nucleotides 238,036 to 238,974 of chromosome IV of Komagataella phaffii CBS 7435, GenBank LT962479.2), the synthetic gene sequence encoding the elastin peptide as described above (SEQ ID NO: 3), and the transcription terminator of the AOX1 gene (SEQ ID NO: 5, nucleotides 240,967 to 241,316 of chromosome IV of Komagataella phaffii CBS 7435, GenBank LT962479.2).

The backbone of the integration plasmid comprises: one drug resistance gene cassette (SEQ ID NO:6) against geneticin (G418), one Col E1 replicon (SEQ ID NO: 7), and one drug resistance gene cassette (SEQ ID NO: 8) against ampicillin. The backbone sequence was synthesized by BGI Tech Solutions (Beijing Liuhe) Co., Ltd. (SEQ ID NO: 9).

To construct the integration plasmid, primers backbone-F and backbone-R (SEQ ID NO: 10 and SEQ ID NO: 11) were used to amplify the backbone sequence (SEQ ID NO: 9).

Genomic DNA of BG11 strain (PS 10011) of BioGrammatics Inc. was prepared using TIANamp Yeast DNA Extraction Kit (DP307-02) from Tiangen Biotech (Beijing) Co., Ltd.

Using the genomic DNA as a template, primers Pro-F and Pro-R (SEQ ID NO: 12 and SEQ ID NO: 13) were used to amplify the methanol-inducible promoter sequence (SEQ ID NO: 4) of the AOX1 gene, and primers Ter-F and Ter-R (SEQ ID NO: 16 and SEQ ID NO: 17) were used to amplify the transcription terminator (SEQ ID NO: 5) of the AOX1 gene.

Using the codon-optimized elastin peptide gene sequence synthesized by BGI Tech Solutions (Beijing Liuhe) Co., Ltd. as a template, primers ELN-F and ELN-R (SEQ ID NO: 14 and SEQ ID NO: 15) were used to amplify the elastin peptide gene (SEQ ID NO: 3).

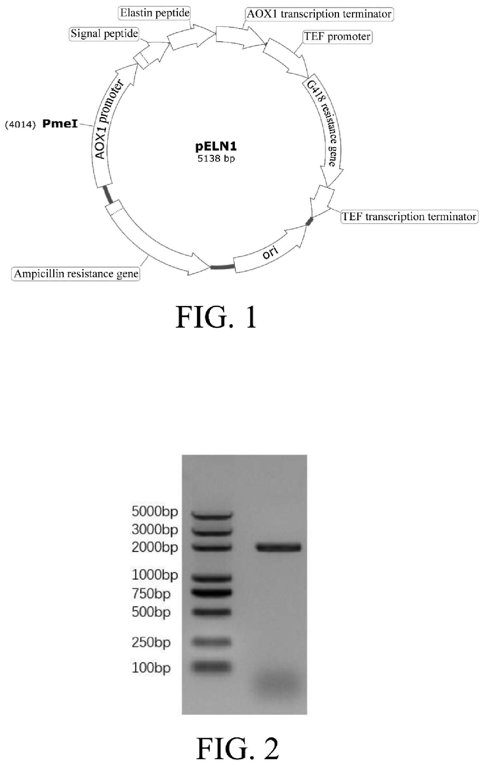

The above amplification products were ligated using NEBuilder HiFi DNA Assembly Premix Kit from NEB. The ligation product was transformed into E. coli DH5α (Shanghai Weidi Biotechnology Co., Ltd., DL1003M). The transformed E. coli clones were screened on Luria-Bertani (LB) medium containing 100 mg/L ampicillin sodium. The assembled sequence of the plasmid was verified by means of Sanger sequencing. The plasmid was designated pELN1 (SEQ ID NO: 18, FIG. 1).

### Construction of an elastin peptide expression strain

The integration plasmid pELN1 was purified from an E. coli culture using the Plasmid Mini Kit (DP103-02) from Tiangen Biotech (Beijing) Co., Ltd. The plasmid was linearized using PmeI restriction enzyme (ThermoFisher ER1341).

100 ng of the linearized plasmid was used to transform a BG11 strain (PS 10011, BioGrammatics Inc.). Preparation of competent cells and electrotransformation were performed according to the protocols in "Expression of proteins in Pichia pastoris" (Methods in Enzymology, 2021 Vol 660, 53-80).

The transformants were screened on YPD solid medium containing 1 g/L G418.

Integration of the integration plasmid into the transformant genome was verified by means of colony PCR using primer 5U (SEQ ID NO: 19), which binds to a genomic sequence upstream of the methanol-inducible promoter of AOX1 gene, and primer 3C (SEQ ID NO: 20), which binds to a plasmid-specific sequence downstream of the elastin expression vector. It was expected that the transformants harboring the integration should produce a PCR product of 2.1 kb (FIG. 2). The PCR product was Sanger sequenced to confirm the presence of the elastin expression vector. The step of colony PCR was also derived from the article "Expression of proteins in Pichia pastoris" (Methods in Enzymology, 2021 Vol 660, 53-80). The verified transformant strain was designated Strain-ELN.

### Protein fermentation process

The fermentation medium (see Table 1) was sterilized at 121°C for 30 minutes and cooled to 28°C for later use, and PTM1 and HMP were added after cooling. The fermentation medium was prepared as shown in Tables 1-2.

**Table 1 Composition of the fermentation medium**

| Reagent | Concentration (g/L) | Brand | Quality grade/Article number |
|---|---|---|---|
| Glycerol | 50 | Natural Oleochemicals Sdn Bhd | Food grade |
| Calcium sulfate dihydrate | 0.46 | Sinopharm Chemical Reagent Co., Ltd. | AR |
| Magnesium sulfate heptahydrate | 5.84 | Sinopharm Chemical Reagent Co., Ltd. | AR |
| Potassium sulfate | 7.34 | Sinopharm Chemical Reagent Co., Ltd. | AR |
| Ammonium sulfate | 9.0 | Sinopharm Chemical Reagent Co., Ltd. | AR |
| Defoamer | 0.2 | Momentive, USA | SAG471 |
| After charging, the mixture was sterilized at 121°C for 30 minutes and cooled to 28°C for later use. | | | |
| PTM1 | Standard PTM1 solution, 4 ml/L HMP (sodium hexametaphosphate solution) | | |
| | 200 g/L standard HMP solution, 84 ml/L | | |

**Table 2 PTM1 formulation**

| Reagent | Concentration (g/L) | Brand | Quality grade/Article number |
|---|---|---|---|
| Copper sulfate pentahydrate | 6 | Sinopharm Chemical Reagent Co., Ltd. | AR |
| Potassium iodide (avoid light during operation) | 0.088 | Sinopharm Chemical Reagent Co., Ltd. | AR |
| Manganese sulfate monohydrate | 3 | Sigma | M7634-500G |
| Sodium molybdate dihydrate | 0.2 | Sinopharm Chemical Reagent Co., Ltd. | AR |
| Boric acid | 0.02 | Sinopharm Chemical Reagent Co., Ltd. | AR |
| Cobalt chloride | 0.5 | Sinopharm Chemical Reagent Co., Ltd. | AR |
| Zinc chloride | 20 | Sinopharm Chemical Reagent Co., Ltd. | AR |
| Ferrous sulfate | 65 | Mackline | F903428-1kg |
| Biotin | 0.2 | Solarbio | D8150 |
| Concentrated sulfuric acid | 5ml | / | / |

The Strain-ELN monoclone was added to 50 mL of the above YPD medium (1% yeast extract powder, 2% peptone, and 2% dextrose), and was cultured overnight at 28°C and 250 rpm (first-stage shake flask seed culture). On the next day, the first stage shake flask seed culture was transferred to 100 mL of the above YPD medium, with the initial OD₆₀₀ being equal to 0.5, and was cultured at 28°C and 250 rpm for 24 h as a seed culture for tank fermentation. The seed culture for tank fermentation was added a fermentation tank containing the described fermentation medium, with the initial OD₆₀₀ being equal to 0.3. Fermentative culturing was performed for 22 h to 24 h, at which time glycerol was added, and the culturing was continued until the wet cell weight was 330 g/L to 380 g/L. Then, the culture was switched to methanol for induction, the induction time being 170 h. The fermentation protocol was performed with reference to the "Pichia Fermentation Process Guidelines" provided by Thermofisher Corp. (https://tools.thermofisher.com/content/sfs/manuals/pichiaferm_prot.pdf).

### Protein purification

The solution of the cultured strain was taken and centrifuged in a low-temperature centrifuge, and the supernatant was retained.

The supernatant obtained above was filtered using a 0.45 µm filter membrane, and then protein purification was performed using a 5 ml nickel ion affinity chromatography column. The procedure was as follows:
1) The sample was flowed through the Ni column at a rate of 1 ml/min;
2) The column was equilibrated using Buffer A (20 mM PB, pH 7.5);
3) Elution was performed using 20 mM, 300 mM, and 500 mM imidazole.
4) The sample portions eluted in the various steps were each subjected to SDS-PAGE gel analysis for the presence of the protein of interest.

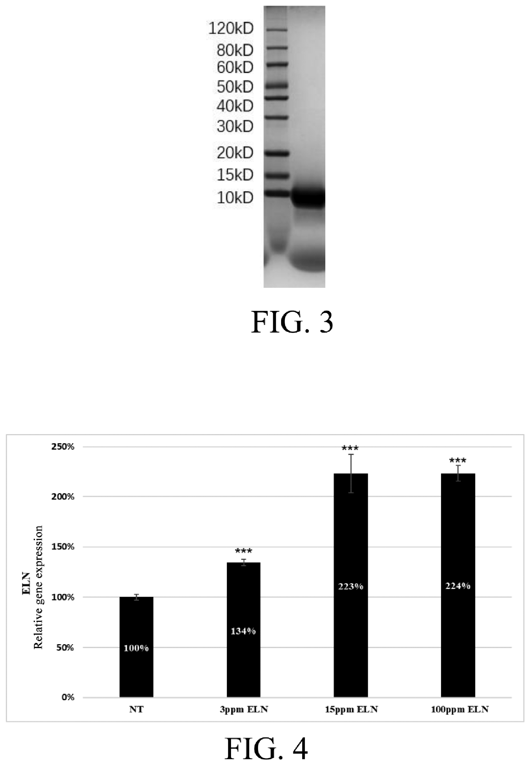

The result is as shown in FIG. 3. It can be seen that the protein of interest was obtained by the method of Example 1.

### Example 2. Formulation with 0.01% of the recombinant human-derived elastin

A formulation was prepared comprising 0.01% of the recombinant human-derived elastin peptide obtained from purification in Example 1, and 99.99% of formulation agents. The formulation agents comprised the following ingredients in percentage by mass: 0.05 parts by weight of a chelating agent, 0.1 parts by weight of a thickener, 3.0 parts by weight of a polyol, 2 parts by weight of a vegetable fat and oil, 1 part by weight of a high-melting point fatty compound, 3 parts by weight of a skin moisturizer, 2 parts by weight of an emulsifier, and 1 part by weight of a preservative, the system being adjusted to a pH of 6.0, and being made up to 100 parts by weight with water.

### Example 3. Formulation with 2% of the recombinant human-derived elastin peptide

A formulation was prepared comprising 2% of the recombinant human-derived elastin peptide obtained from purification in Example 1, and 98% of formulation agents. The formulation agents comprised the following ingredients in percentage by mass: 0.5 parts by weight of a chelating agent, 0.2 parts by weight of a thickener, 4.0 parts by weight of a polyol, 3 parts by weight of a vegetable fat and oil, 2 parts by weight of a high-melting point fatty compound, 8 parts by weight of a skin moisturizer, 6 parts by weight of an emulsifier, and 2 part by weight of a preservative, the system being adjusted to a pH of 6.5, and being made up to 100 parts by weight with water.

### Example 4. Formulation with 5% of the recombinant human-derived elastin peptide

A formulation was prepared comprising 5% of the recombinant human-derived elastin peptide obtained from purification in Example 1, and 95% of formulation agents. The formulation agents comprised the following ingredients in percentage by mass: 1 part by weight of a chelating agent, 0.5 parts by weight of a thickener, 6.0 parts by weight of a polyol, 5 parts by weight of a vegetable fat and oil, 3 parts by weight of a high-melting point fatty compound, 20 parts by weight of a skin moisturizer, 10 parts by weight of an emulsifier, and 3 part by weight of a preservative, the system being adjusted to a pH of 7.0, and being made up to 100 parts by weight with water.

In the described Examples 2-4, the chelating agent could be EDTA 2Na, the thickener could be Carbomer or xanthan gum, the polyol could be glycerol or pentanediol, the vegetable fat was shea butter, the high-melting-point fatty compound could be cetearyl alcohol, cetyl ethylhexanoate, cetearyl alcohol or cetyl ethylhexanoate, the skin moisturizer could be a cetearyl glucoside mixture, the emulsifier could be glyceryl stearate, a PEG-100 stearate mixture or triethylhexanoin, and the preservative could be p-hydroxyacetophenone.

### Example 5. Verification of the efficacy of the recombinant human-derived elastin peptide

### Method for testing gene expression:

Primary human-derived fibroblasts (HDFs) were purchased from Lifeline^{®} Cell Technology, and were cultured under conditions of 95% air, 5% CO₂, and 37°C. On reaching 80% to 90% density, the HDFs were digested from the medium and seeded into a 12-well plate at a concentration of 100,000 cells/well. After 48 hours, the cells were treated with different concentrations (3 ppm, 15 ppm, and 100 ppm) of the test sample (the recombinant elastin peptide obtained from purification in Example 1) (n = 3), and the HDFs were exposed to UVA irradiation at 5 J/cm2 for 24 hours. After 24 hours of treatment, the HDFs were rinsed twice with a cooled PBS buffer, and then total RNA was extracted from the cell lysate using Qiagen's RNeasy Mini Kit. Total RNA was reverse transcribed into cDNA for amplification. The expression of the gene of interest was determined by quantitative PCR (qPCR) (primers are as shown in Table 3). The relative gene expression level was calculated by means of a 2-ΔΔCt method.

**Table 3 Primers for determining the gene of interest by quantitative PCR**

| Gene | Primer name | Sequence |
|---|---|---|
| ELN | Forward | GGTTGTGTCACCAGAAGCAGCT (SEQ ID NO: 32) |
| | Reverse | CCGTAAGTAGGAATGCCTCCAAC (SEQ ID NO: 33) |
| COL7A1 | Forward | GTTGGAGAGAAAGGTGACGAGG (SEQ ID NO: 34) |
| | Reverse | TGGTCTCCCTTTTCACCCACAG (SEQ ID NO: 35) |
| COL4A1 | Forward | TGTTGACGGCTTACCTGGAGAC (SEQ ID NO: 36) |
| | Reverse | GGTAGACCAACTCCAGGCTCTC (SEQ ID NO: 37) |
| COL1A1 | Forward | GATTCCCTGGACCTAAAGGTGC (SEQ ID NO: 38) |
| | Reverse | AGCCTCTCCATCTTTGCCAGCA (SEQ ID NO: 39) |
| Internal reference YWHAZ | Forward | TGATCCCCAATGCTTCACAAG (SEQ ID NO: 40) |
| | Reverse | GCCAAGTAACGGTAGTAATCTCC (SEQ ID NO: 41) |

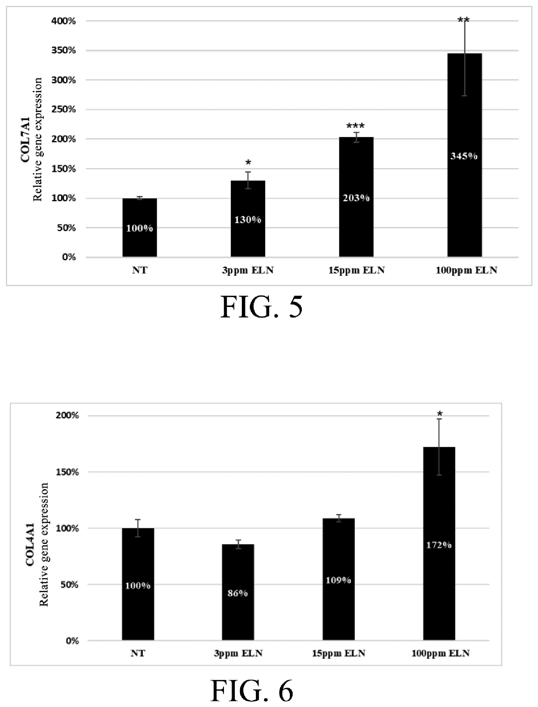

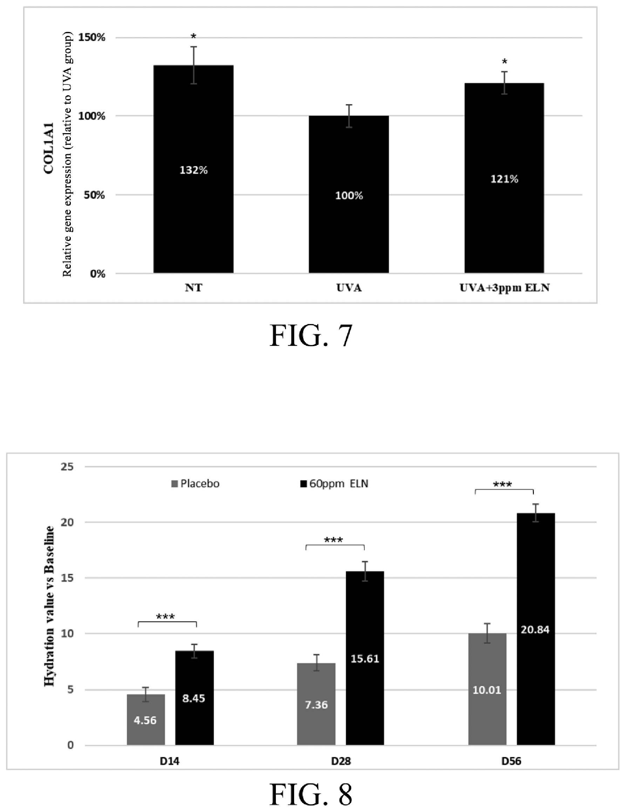

FIG. 4 is a graph showing the promoting effect of the recombinant elastin peptide on the expression of elastin gene in fibroblasts, wherein, as the concentration of the recombinant elastin (ELN) added increased, the gene expression of elastin significantly increased and reached a high value at 15 ppm. FIG. 5 is a graph showing the promoting effect of the recombinant elastin peptide on the gene expression of collagen VII (COL7A1), wherein, as the concentration of the recombinant elastin (ELN) added increased, the gene expression of collagen VII significantly increased in a concentration-dependent manner. FIG. 6 is a graph showing the promoting effect of the recombinant elastin peptide on the gene expression of collagen IV (COL4A1), wherein, as the concentration of the recombinant elastin (ELN) added increased, the gene expression of collagen IV significantly increased in a concentration-dependent manner. FIG. 7 is a comparison graph of UVA irradiation, wherein the gene expression of collagen I in fibroblasts after UVA irradiation was significantly decreased, whereas, in the fibroblasts treated with the recombinant elastin (ELN), the gene expression of collagen I was restored, demonstrating that the recombinant elastin had significant protection efficacy against UVA.

### Method for testing clinical efficacy:

Thirty five healthy female volunteers aged 30 to 60 years (whose skin conditions satisfied any two of the wrinkle conditions of crow's feet of grade 2 to 4, under-eye fine wrinkles of grade 2 to 5, and nasolabial folds of grade 1 to 3, and also satisfied an F4 mean value of greater than 6 or an R2 mean value of less than or equal to 0.65 for both the left cheek and the right cheek) used an emulsion formulation containing 2% of the recombinant elastin (i.e., the product of Example 3) and a placebo formulation (the placebo formulation differed from the product of Example 3 in that water was used to replace the recombinant elastin) continuously for 56 days. The placebo and sample (i.e., the emulsion formulation containing 2% of the recombinant elastin) groups were tested randomly, with the placebo and sample being applied to half of the face, twice daily. On D0, D14, D28 and D56, the moisture content of the skin was measured using a CM825 (CK) instrument, and indexes such as skin color, skin gloss and crow's feet were each measured using a VISIA-CR instrument, thereby analyzing the effect of the sample on skin condition.

FIG. 8 is a comparison graph of skin moisture content after 14 days of use of an emulsion formulation comprising 60 ppm of the recombinant elastin peptide, wherein 2% ELN (i.e., the product of Example 4 of the present invention) achieved a skin moisture content 100% higher than that of the control group (placebo), and the effect was more significant as the use time increased.

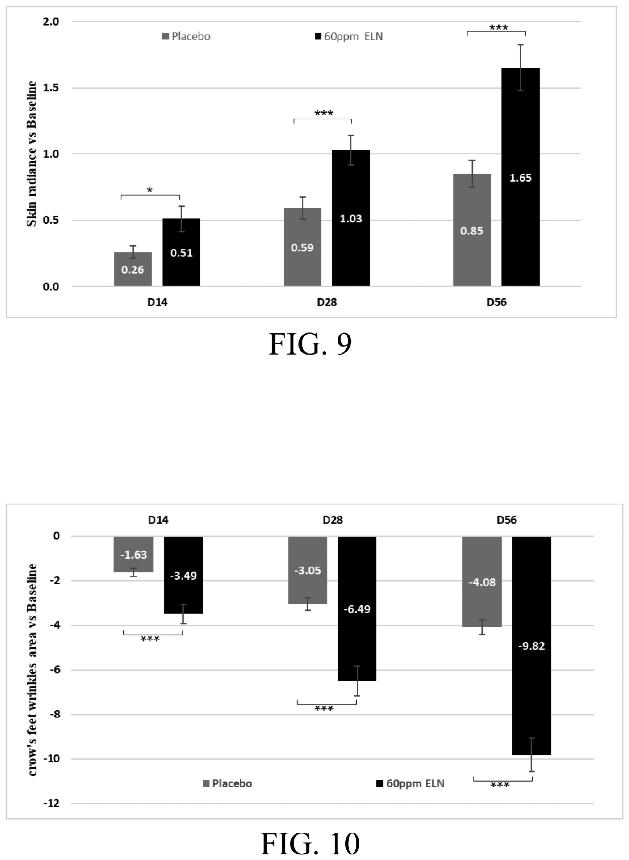

FIG. 9 is a comparison graph comparing skin gloss after 14 days of use of an emulsion formulation comprising 60 ppm of the recombinant elastin peptide, wherein 2% ELN (i.e., the product of Example 3 of the present invention) achieved a significant increase in skin gloss, and as the use time increased, i.e., after two months, the skin gloss could achieve a two-fold improvement.

FIG. 10 is a comparison graph of crow's feet after 14 days of use of an emulsion formulation comprising 60 ppm of the recombinant elastin peptide, wherein 2% ELN (i.e., the product of Example 3 of the present invention) had a very significant effect in reducing crow's feet wrinkles, and as the use time increased, the effect was more obvious.

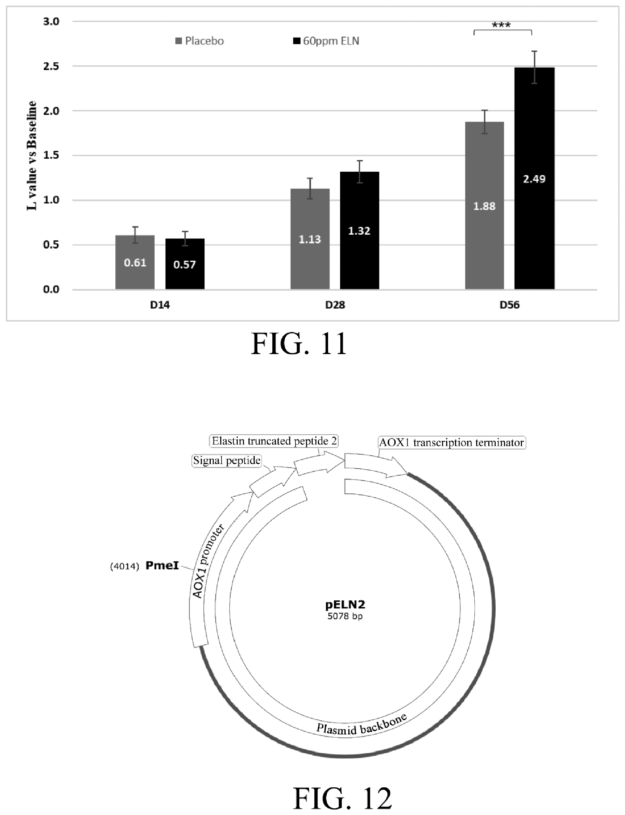

FIG. 11 is a comparison graph of skin L value after two months of use of an emulsion formulation comprising 60 ppm of the recombinant elastin peptide, wherein 2% ELN (i.e., the product of Example 3 of the present invention) achieved a significant increase in the skin L value in comparison to the control group (placebo), indicating that the formulation had a certain whitening efficacy.

### Example 6. Construction of an expression system of the recombinant human-derived elastin peptide

An expression system of the recombinant human-derived elastin peptide having the sequence of SEQ ID NO: 21 was further constructed. The construction method was as follows: the design of the C-terminal His tag in the expression plasmid pELN1 disclosed in Example 1 was retained, i.e. the C-terminus of SEQ ID NO: 21 carried the His tag (-GSSHHHHHH) of SEQ ID NO: 30, with the sequence of the recombinant human-derived elastin peptide-His tag fusion protein being SEQ ID NO:29. Using pELN1 (SEQ ID NO: 18) as a template, primers ELN2-F (SEQ ID NO: 22) and ELN2-R (SEQ ID NO: 23) were used to PCR amplify the coding sequence of the recombinant human-derived elastin peptide (SEQ ID NO: 31). Using pELN as a template, primers BB-F (SEQ ID NO: 24) and BB-R (SEQ ID NO: 25) were used to PCR amplify a plasmid backbone, the plasmid backbone comprising the coding sequences for the AOX1 promoter, Saccharomyces cerevisiae mating factor α signal peptide, AOX1 transcription terminator, geneticin (G418) drug resistance gene cassette, Col E1 replicon, and ampicillin drug resistance gene cassette.

The above amplification products were ligated using NEBuilder HiFi DNA Assembly Premix Kit from NEB. The ligation product was used to transform E. coli DH5α (Shanghai Weidi Biotechnology Co., Ltd., DL1003M). The transformed E. coli clones were screened on Luria-Bertani (LB) medium containing 100 mg/L ampicillin sodium. The assembled sequence of the plasmid was verified by means of Sanger sequencing. The plasmid was designated pELN2 (Seq ID NO: 26, FIG. 12).

The integration plasmid pELN2 was purified from an E. coli culture using the Plasmid Mini Kit (DP103- 02) from Tiangen Biotech (Beijing) Co., Ltd. The plasmid was linearized using PmeI restriction enzyme (ThermoFisher ER1341).

100 ng of the linearized plasmid was used to transform a BG11 strain (PS10011, BioGrammatics Inc.). Preparation of competent cells and electrotransformation were performed according to the protocols in "Expression of proteins in Pichia pastoris" (Methods in Enzymology, 2021 Vol 660, 53-80).

The transformants were screened on YPD solid medium containing 1 g/L G418.

Integration of the integration plasmid into the transformant genome was verified by means of colony PCR using primer 5U2 (SEQ ID NO: 27), which binds to a genomic sequence upstream of the methanol-inducible promoter of AOX1 gene, and primer 3C2 (SEQ ID NO: 28), which binds to the elastin peptide coding sequence. It was expected that the transformants harboring the integration should produce a PCR product of 1.4 kb. The PCR product was Sanger sequenced to confirm the presence of the elastin expression vector. The step of colony PCR was also derived from the article "Expression of proteins in Pichia pastoris" (Methods in Enzymology, 2021 Vol 660, 53-80). The verified transformant strain was designated Strain-ELN2.

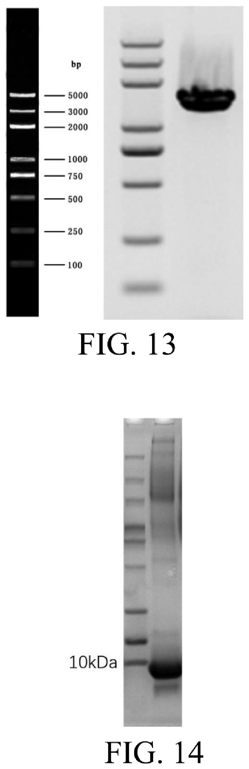

FIG. 13 is an electrophoretic image showing insertion of the recombinant human-derived elastin peptide integration plasmid pELN2 into the genome of a transformant as verified by PCR in an example of the present invention.

The fermentation process and protein purification process of the protein were the same as in Example 1. The fermentation product was determined by SDS-Page. The result is as shown in FIG. 14.

### Example 7. Test of clinical efficacy

Healthy female volunteers aged 30 to 60 years (whose skin conditions satisfied any two of the wrinkle conditions of crow's feet of grade 2 to 4, under-eye fine wrinkles of grade 2 to 5, and nasolabial folds of grade 1 to 3, and also satisfied an F4 mean value of greater than 6 or an R2 mean value of less than or equal to 0.65 for both the left cheek and the right cheek) were selected, and used an emulsion formulation containing 60 ppm of the recombinant elastin and a placebo formulation continuously for 56 days. The placebo and sample groups were tested randomly, with the placebo and sample being applied to half of the face, twice daily. The dermis density, skin elasticity, and skin firmness of the volunteers after 14, 28, and 56 days of use were determined using a VISIA-CR instrument.

The emulsion formulation used the recombinant human-derived elastin peptide (SEQ ID NO: 29) obtained from purification in Example 6, and the emulsion was formulated according to the formula of Example 3. The placebo formulation was prepared by replacing the recombinant human-derived elastin in the emulsion with water.

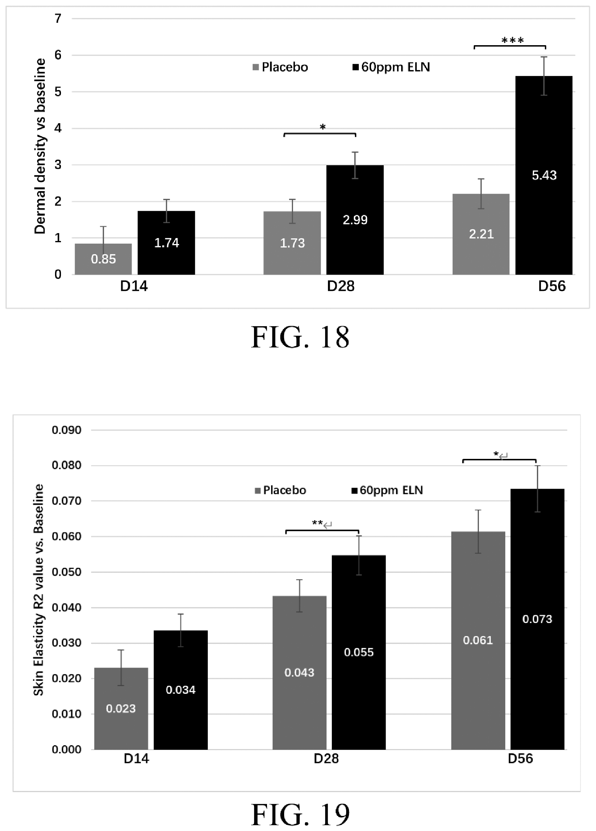

FIG. 18 is a comparison diagram of dermal density at 14 days, 28 days and 56 days of use of the emulsion formulation containing 60 ppm of the recombinant elastin peptide of the present invention (compared with the control group). The skin density could be significantly increased after just 14 days of using the emulsion containing 60 ppm of the recombinant elastin, and the effect increased as use time increased. After 28 and 56 days of use, the increase in skin density was significantly higher than that of the control group.

FIG. 19 is a comparison diagram of skin elasticity at 14 days, 28 days and 56 days of use of the emulsion formulation containing 60 ppm of the recombinant elastin peptide of the present invention (compared with the control group). In the case of using the emulsion containing 60 ppm of the recombinant elastin, skin elasticity gradually increased over time, as indicated by a numerical value superior to that of the control group.

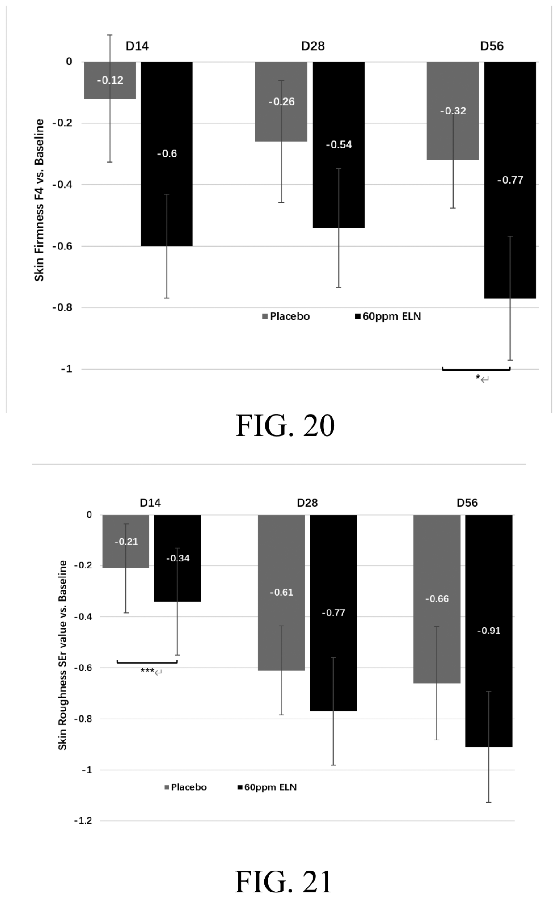

FIG. 20 is a comparison diagram of skin firmness at 14 days, 28 days and 56 days of use of the emulsion formulation containing 60 ppm of the recombinant elastin peptide of the present invention (compared with the control group). In the case of using the emulsion containing 60 ppm of the recombinant elastin, skin firmness increased as use time increased, and the effect was far superior to that of the control group.

FIG. 21 is a comparison diagram of skin roughness at 14 days, 28 days and 56 days of use of the emulsion formulation containing 60 ppm of the recombinant elastin peptide of the present invention (compared with the control group). In the case of using the emulsion containing 60 ppm of the recombinant elastin, the improvement in skin roughness increased over time, with the data after 14 days being superior to that of the control group.

### Example 8. Study of the skin permeability of the recombinant human-derived elastin peptide

The recombinant human-derived elastin peptide had a sequence containing 89 amino acids (SEQ ID NO: 29) and a molecular weight of about 8 Kd. In order to verify that the recombinant human-derived elastin peptide possessed transdermal properties, the inventors used FITC to label the recombinant human-derived elastin peptide, and used an artificially constructed 3D skin model (EpiKutis) to perform transdermal experimentation and discovery.

### 8.1 Fluorescent labeling of the recombinant human-derived elastin peptide

The recombinant human-derived elastin peptide was dissolved in a sodium carbonate buffer, and an FITC solution was slowly added dropwise. After the addition of the required FITC was completed, the reaction solution was incubated at 4°C in darkness for 8 h. An NH₄Cl solution (1 M) was added to the reaction solution, and the mixture was gently shaken evenly to terminate the reaction at 4°C for 2 h.

The above labeled substance was separated by chromatography using a Sephadex G25 filtration chromatography column. After the gel chromatography column was equilibrated with 5 to 10 column volumes of PBS (10 mM, pH 7.4), 1 mL of the reaction mixture was injected from the top of the column. The gel column was opened, and after all of the reaction mixture flowed into the column bed, the sample was washed three times with a small amount of PBS buffer (10 mM, pH 7.4), and then eluted with added PBS buffer (10 mM, pH 7.4). Two chromatographic bands appeared in the elution, which were FITC-recombinant human-derived elastin peptide and FITC, from bottom to top.

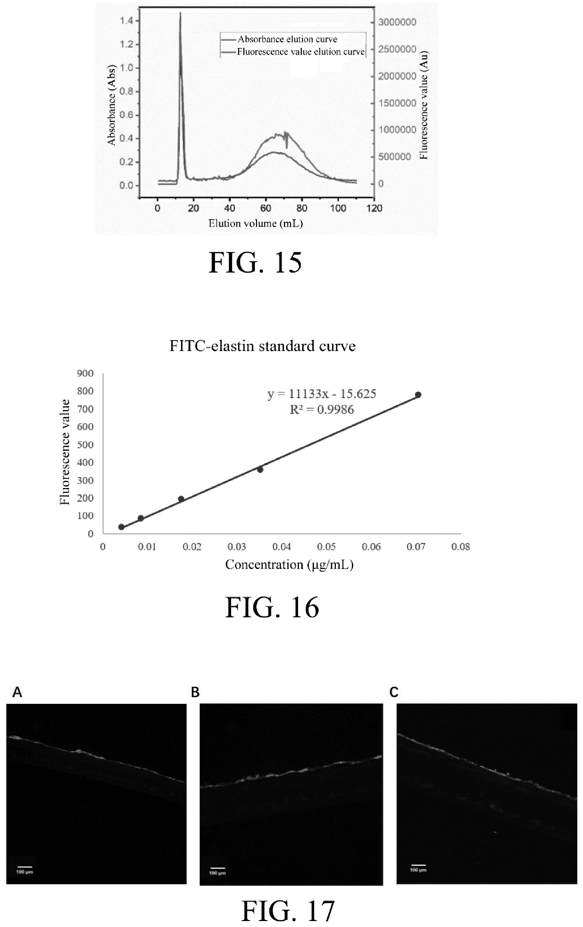

One column volume of sample was collected to plot an elution curve. The eluent was collected at 0.5 mL/tube, and a total of 220 tubes were collected. The absorbance and fluorescence value at 495 nm (excitation wavelength at 495 nm, and emission wavelength at 525 nm) were measured for each tube. The elution curve was plotted using elution volume as the abscissa and absorbance/fluorescence value as the ordinate (FIG. 15). The results revealed that elution curves plotted using absorbance and fluorescence value, respectively, substantially overlapped and showed two elution peaks. The first peak at 10 mL to 15 mL was FITC-elastin, and the second peak eluted after 40 mL was free FITC. The two elution peaks were completely separate, indicating that the FITC-elastin was successfully separated and purified. The eluent at 10 mL to 15 mL was collected and freeze dried, to obtain a solid FITC-recombinant human-derived elastin peptide conjugate.

### 8.2 Preparation of samples to be tested

An emulsion and an aqueous agent of the recombinant human-derived elastin peptide were prepared according to the formulas in Tables 4 and 5, respectively.

**Table 4 Elastin peptide emulsion**

| Elastin peptide emulsion | | | | |
|---|---|---|---|---|
| Phase | Trade Name | INCI Name | % | Function |
| | Water | Water | to 100 | Solvent |
| A | Dissolvine Na2 | Disodium EDTA | 0.05 | Chelating agent |
| | Carbopol 980 Polymer | Carbomer | 0.05 | Thickener |
| | SEPIMAX ZEN^{™} | Polyacrylate crosspolymer-6 | 0.2 | Thickener |
| | Xanthan Gum FN Translucent xanthan gum | Xanthan gum | 0.05 | Thickener |
| B | Glucate SS | Methyl glucose sesquistearate | 0.2 | Emulsifier |
| | Glucamate^{™} SSE-20 | PEG-20 methyl glucoside sesquistearate | 0.5 | Emulsifier |
| | 100 viscosity silicone oil | Polydimethylsiloxane | 3 | Emollient |
| | GLTEST TGB | Triethylhexanoin | 1 | Emollient |
| C | NaOH (10%) | Sodium hydroxide, water | 0.1 | pH regulator |
| D | Phytol | Phytol | 1 | Penetration enhancer |
| | 1,3-propanediol | Propylene glycol | 5 | Humectant |
| | Sabinsa | Tetrahydropiperine | 0.2 | Penetration enhancer |
| | FITC-elastin peptide | Elastin peptide | 0.02 | Test sample |
| | Tridecapeptide-1 | Tridecapeptide-1 | 0.01 | Penetration enhancer |

**Table 5 Recombinant elastin aqueous agent**

| Elastin aqueous agent | | | | |
|---|---|---|---|---|
| Phase | Trade Name | INCI Name | % | Function |
| | Water | Water | to 100 | Solvent |
| | FITC-elastin peptide | Elastin | 0.02 | Test sample |
| | 1,3-propanediol | Propylene glycol | 5 | Humectant |
| A | Sabinsa | Tetrahydropiperine | 0.1 | Penetration enhancer |
| | Tridecapeptide-1 | Tridecapeptide-1 | 0.01 | Penetration enhancer |
| | CO40 | PEG-40 hydrogenated castor oil | 0.2 | Solubilizer |

### 8.3 Resuscitation and culture of the 3D skin model

The constructed 3D skin (EpiKutis) was placed in a 6-well plate containing 0.9 mL of culture solution, and cultured in a CO₂ incubator for 1 hour. The 3D skin was transferred to a 6-well plate containing 0.9 mL of EpiGrowth culture solution (Guangdong BioCell Biotechnology Co., Ltd.), and cultured in a CO₂ incubator for 18 hours before carrying out the study.

### 8.4 Procedure and results of the transdermal experiment

An FITC-recombinant human-derived elastin peptide aqueous solution at a concentration of 0.2 mg/mL, an FITC-recombinant human-derived elastin peptide emulsion at a concentration of 0.2 mg/mL (see Table 4), and an FITC-recombinant human-derived elastin peptide aqueous agent at a concentration of 0.2 mg/mL (see Table 5) were each prepared. After adding 25 µL of each sample to the surface of the 3D skin in a clean bench, the 3D skin was placed in a CO₂ incubator to investigate permeation through the 3D skin using 0.9 mL of culture medium (EpiGrowth culture solution) as a receiving solution. 200 µL of the receiving solution was taken at each of 0.25, 0.5, 1, 2, 4, 6, 8, 10 and 12 h to perform fluorescence determination, and at the same time, an equal volume of blank medium (EpiGrowth culture solution) at the same temperature was added for replenishment. The fluorescence value of each sample was determined using a microplate reader (excitation wavelength at 495 nm, and emission wavelength at 530 nm). The fluorometric quantification standard curve was as shown in FIG. 16.

**Table 6 Transdermal data of the samples in the 3D skin model**

| Sample | | Time (h) | | | | | | | | |
|---|---|---|---|---|---|---|---|---|---|---|
| | | 0.25 | 0.5 | 1 | 2 | 4 | 6 | 8 | 10 | 12 |
| Recombinant human-derived elastin peptide aqueous solution | Cumulative permeation amount (ng) | / | / | / | / | / | / | 5.5 | 6.3 | 13.3 |
| | Cumulative permeation percentage (%) | / | / | / | / | / | / | 0.11 | 0.13 | 0.27 |
| Recombinant human-derived | Cumulative permeation amount (ng) | / | / | / | / | / | / | / | / | 6.0 |
| elastin peptide emulsion | Cumulative permeation percentage (%) | / | / | / | / | / | / | / | / | 0.12 |
| Recombinant human-derived elastin peptide aqueous agent | Cumulative permeation amount (ng) | / | / | / | / | / | / | 5.2 | 8.4 | 10.8 |
| | Cumulative permeation percentage (%) | / | / | / | / | / | / | 0.11 | 0.17 | 0.22 |

The determination results of each sample in the 3D skin model can be seen in Table 6. The elastin aqueous solution and the recombinant human-derived elastin peptide aqueous agent had better permeation performance than the recombinant human-derived elastin peptide emulsion; for the former two, the fluorescence signal of FITC-elastin was detected in the receiving cell after 8 h of administration, and the permeation amount was about 0.2% after 12 h. For the recombinant human-derived elastin peptide emulsion, the fluorescence signal of FITC-elastin was consistently lower than the limit of quantitation in the determinations performed at or before 10 h of administration, and the cumulative permeation amount was 0.12% after 12 h.

### 8.5 Observation under confocal laser scanning microscope (CLSM)

According to the foregoing method for investigation of permeation through the 3D skin model, the 3D skin was treated with each of the three samples, i.e., the recombinant human-derived elastin peptide aqueous solution, the recombinant human-derived elastin peptide emulsion, and the recombinant human-derived elastin peptide aqueous agent, for 12 h. Then, the 3D skin was subjected to cryosectioning and DAPI staining, and the distribution of FITC-elastin in the skin was determined using a CLSM (FIG. 17A-C). The fluorescence signal of each sample was mainly concentrated in the stratum corneum, and 12 hours after treatment of the 3D skin with each sample, there was a slight fluorescence signal in the epidermal layer of the 3D skin, with the fluorescence signal of the elastin aqueous agent being the strongest (see FIG. 17C), indicating that the recombinant human-derived elastin peptide of this dosage form had the best penetration effect.

Notwithstanding the foregoing, those skilled in the art should understand that the present invention is not limited by the above examples, and that the above examples and the description are only intended to explain the principle of the present invention. The present invention is amenable to various variations and modifications without departing from the spirit and scope of the present invention, and those variations and modifications all fall within the scope of the present invention as claimed. The scope of the present invention as claimed is defined by the appended claims and equivalents thereof.

## Claims

1. A recombinant human-derived elastin peptide having anti-aging efficacy, the recombinant human-derived elastin peptide being selected from the following a) to c): a) comprising the amino acid sequence set forth in SEQ ID NO: 1; b) comprising a fragment of the amino acid sequence set forth in SEQ ID NO: 1, the fragment comprising 30% or more of SEQ ID NO: 1; and c) mutants of a) and b) above.

2. The recombinant human-derived elastin peptide according to claim 1, wherein the recombinant human-derived elastin peptide comprises the amino acid sequence set forth in SEQ ID NO: 21, or comprises variant sequences obtained by insertion, substitution or deletion of one or more amino acids on the basis of SEQ ID NO: 21; the variant sequences having at least 90% or more identity to SEQ ID NO: 21; preferably, the variants retaining an anti-aging efficacy equivalent to that of SEQ ID NO: 21; preferably, the anti-aging efficacy being one or more selected from the following: 1) promoting the expression of elastin per se; 2) promoting gene expression of collagen IV and/or VII in the dermal-epidermal junction layer; 3) protecting collagen I from being damaged by ultraviolet rays; 4) increasing or maintaining the water content of skin; 5) improving skin gloss; 6) reducing crow's feet; 7) increasing the L value of skin; 8) increasing skin density; and 9) increasing skin elasticity or firmness.

3. The recombinant human-derived elastin peptide having anti-aging efficacy according to claim 1 or 2, wherein the N-terminus and/or C-terminus of the sequence of the recombinant human-derived elastin peptide comprises a fusion protein sequence.

4. The recombinant human-derived elastin peptide having anti-aging efficacy according to claim 3, wherein the sequence of the recombinant human-derived elastin peptide comprises a protein tag at the N-terminus and/or C-terminus.

5. The recombinant human-derived elastin peptide having anti-aging efficacy according to claim 4, wherein the protein tag is one of a His tag, a glutathione thioltransferase tag, a maltose binding protein tag, a SUMO tag, a NusA tag, a TrxA tag, and a DsbA tag; preferably, the recombinant human-derived elastin peptide comprises the amino acid sequence set forth in SEQ ID NO: 29.

6. The recombinant human-derived elastin peptide having anti-aging efficacy according to claim 1, wherein the recombinant human-derived elastin peptide is expressed by a host.

7. The recombinant human-derived elastin peptide having anti-aging efficacy according to claim 6, wherein the recombinant human-derived elastin peptide is expressed by a bacterial host.

8. The recombinant human-derived elastin peptide having anti-aging efficacy according to claim 7, wherein the bacterial host is one of Escherichia coli, Bacillus subtilis, and Rhodococcus erythropolis.

9. The recombinant human-derived elastin peptide having anti-aging efficacy according to claim 6, wherein the recombinant human-derived elastin peptide is expressed by a fungal host.

10. The recombinant human-derived elastin peptide having anti-aging efficacy according to claim 9, wherein the fungal host is one of Pichia pastoris, Saccharomyces cerevisiae, and Aspergillus oryzae.

11. The recombinant human-derived elastin peptide having anti-aging efficacy according to claim 6, wherein the recombinant human-derived elastin peptide is expressed by a eukaryotic cell host.

12. The recombinant human-derived elastin peptide having anti-aging efficacy according to claim 11, wherein the eukaryotic cell is one of an insect cell, a CHO cell, a mouse cell, and a human cell.

13. The recombinant human-derived elastin peptide having anti-aging efficacy according to claim 6, wherein the recombinant human-derived elastin peptide is expressed in a host cell.

14. The recombinant human-derived elastin peptide having anti-aging efficacy according to claim 6, wherein the recombinant human-derived elastin peptide is expressed and secreted by a host cell.

15. The recombinant human-derived elastin peptide having anti-aging efficacy according to claim 14, wherein the recombinant human-derived elastin peptide is expressed and secreted, and a precursor protein thereof includes a secretory signal peptide sequence.

16. The recombinant human-derived elastin peptide having anti-aging efficacy according to claim 14, wherein the recombinant human-derived elastin peptide is expressed and excreted by one of Pichia pastoris or Saccharomyces cerevisiae.

17. The recombinant human-derived elastin peptide having anti-aging efficacy according to claim 15, wherein a precursor protein thereof comprises a signal peptide sequence of Saccharomyces cerevisiae mating factor α, the amino acid sequence of the signal peptide of the mating factor α being as shown in SEQ ID NO: 2.

18. The recombinant human-derived elastin peptide having anti-aging efficacy according to claim 1 or 2, wherein the gene encoding the recombinant human-derived elastin peptide comprises a fragment encoding the elastin peptide in the cDNA sequence of the human-derived elastin gene.

19. The recombinant human-derived elastin peptide having anti-aging efficacy according to claim 1 or 2, wherein the gene encoding the recombinant human-derived elastin peptide is an artificially synthesized gene.

20. The recombinant human-derived elastin peptide having anti-aging efficacy according to claim 15, wherein the recombinant human-derived elastin peptide is expressed and secreted, and the gene thereof encodes a precursor of the recombinant human-derived elastin peptide, as represented by the fact that the gene thereof comprises a nucleotide sequence encoding the signal peptide and a nucleotide sequence encoding the recombinant human-derived elastin peptide.

21. The recombinant human-derived elastin peptide having anti-aging efficacy according to claim 20, wherein the gene sequence encoding the recombinant human-derived elastin peptide is a codon-optimized artificially synthesized gene sequence.

22. The recombinant human-derived elastin peptide having anti-aging efficacy according to claim 15, wherein the recombinant human-derived elastin peptide is expressed and secreted, and the gene thereof encodes a precursor of the recombinant human-derived elastin peptide, as represented by the fact that the gene thereof comprises a nucleotide sequence encoding the signal peptide, a nucleotide sequence encoding the recombinant human-derived elastin peptide, and a nucleotide sequence encoding a C-terminal His tag.

23. The recombinant human-derived elastin peptide having anti-aging efficacy according to claim 22, wherein the artificially synthesized gene encoding the elastin peptide is obtained by performing codon optimization on a nucleotide sequence encoding the signal peptide, the elastin peptide and the C-terminal His tag, and then performing synthesis.

24. The recombinant human-derived elastin peptide having anti-aging efficacy according to claim 23, wherein the artificially synthesized gene encoding the elastin peptide comprises a nucleotide sequence as shown in SEQ ID NO: 3 or 31.

25. An isolated nucleic acid, encoding the recombinant human-derived elastin peptide according to any one of claims 1-24.

26. A vector, comprising the isolated nucleic acid according to claim 25.

27. A host cell, comprising the nucleic acid according to claim 25 or the vector according to claim 26.

28. A method for preparing the recombinant human-derived elastin peptide having anti-aging efficacy according to any one of claims 1 to 24, comprising: initiating expression of the gene encoding the recombinant human-derived elastin peptide or a precursor protein thereof by means of a promoter; the promoter being an inducible promoter or a constitutive promoter.

29. A method for preparing the recombinant human-derived elastin peptide having anti-aging efficacy according to any one of claims 1-24 from a fermentation culture, comprising the following steps:
1) performing fermentation of a host cell comprising the gene encoding the recombinant human-derived elastin peptide or a precursor protein thereof, to obtain a fermentation culture; preferably, the host cell being as described in claim 27; and
2) performing protein purification, to obtain the recombinant human-derived elastin peptide.

30. The method for preparing the recombinant human-derived elastin peptide having anti-aging efficacy from a fermentation culture according to claim 29, wherein, for a fermentation culture involving intracellular expression, the recombinant human-derived elastin peptide is prepared from a cell lysate; and for a fermentation culture involving expression and secretion, the recombinant human-derived elastin peptide is prepared from a fermentation supernatant;
preferably, the cell lysate or fermentation supernatant is filtered using a 0.45 µm filter membrane, and then protein purification is performed using an affinity chromatography column, to obtain the recombinant human-derived elastin peptide having anti-aging efficacy.

31. A composition of a recombinant human-derived elastin peptide having anti-aging efficacy, the composition comprising the recombinant human-derived elastin peptide according to any one of claims 1-24 in an amount of 0.01% to 5%, the remainder being formulation agents.

32. A composition of a recombinant human-derived elastin peptide having anti-aging efficacy, the composition comprising the recombinant human-derived elastin peptide and auxiliary agents; preferably, the recombinant human-derived elastin peptide comprising the amino acid sequence shown in SEQ ID NO: 21; preferably, the composition being an emulsion, an aqueous agent or an aqueous solution.

33. The composition according to claim 32, wherein the composition is an aqueous agent, and the auxiliary agents comprise a solvent, a humectant, a penetration enhancer, and a solubilizer; preferably, the solvent is water; preferably, the humectant comprises propylene glycol; preferably, the penetration enhancer comprises tetrahydropiperine and/or tridecapeptide-1; and preferably, the solubilizer comprises PEG-40 hydrogenated castor oil.

34. The composition according to claim 32, wherein the composition is an emulsion, and the auxiliary agents comprise one or more selected from the group consisting of a solvent, a chelating agent, a thickener, an emulsifier, an emollient, a humectant, a penetration enhancer, and a pH regulator. preferably, the solvent is water; preferably, the chelating agent is disodium EDTA; preferably, the thickener comprises one or more of Carbomer, xanthan gum and polyacrylate crosspolymer-6; preferably, the emulsifier is selected from methyl glucose sesquistearate and/or PEG-20 methyl glucoside sesquistearate; preferably, the emollient is selected from dimethicone and/or triethylhexanoin; preferably, the penetration enhancer is selected from one or more of phytol, tetrahydropiperine, and tridecapeptide-1; and preferably, the humectant comprises propylene glycol.

35. A use of the recombinant human-derived elastin peptide having anti-aging efficacy according to any one of claims 1-24, or the composition according to any one of claims 31-34, in the preparation of skin care products, skin repair dressings, implants, artificial skin, medical devices, and/or biomaterials.
